# EUROPEAN PATENT APPLICATION

(11) **EP 4 772 161 A1**
(43) Date of publication of application: **08.07.2026**
(21) Application number: 24859861.7
(22) Date of filing: 28.08.2024
(51) Int. Cl.: A61K 8/31, A61K 8/02, A61K 8/04, A61Q 1/00, A61Q 1/06, A61Q 1/12, A61Q 1/14, A61Q 19/00, A61Q 19/08, A61Q 19/10

(54) **SKIN COSMETIC COMPOSITION CONTAINING BOTRYOCOCCENE AS ACTIVE INGREDIENT**

(30) Priority: 28.08.2023 JP 2023138134
(71) Applicant: Phycochemy Corporation, Tsukuba-shi, Ibaraki 300-2646 (JP)
(72) Inventor: KUROKAWA, Hiromi, Tsukuba-shi, Ibaraki 300-2646 (JP); ATSUMI, Kinya, Tsukuba-shi, Ibaraki 300-2646 (JP); WATANABE, Makoto, Tsukuba-shi, Ibaraki 300-2646 (JP)
(74) Representative: AWA Sweden AB
(86) International application number: PCT/JP2024/030826
(87) International publication number: WO 2025/047832

(57) **Abstract**

It is an object of the present invention to provide a skin cosmetic composition comprising a botryococcene as an active ingredient, for prevention or alleviation of undesirable cosmetic changes in the skin. By multiple tests it was confirmed that botryococcenes inhibit ROS production and its downstream effects which produce undesirable cosmetic changes in skin, leading to discovery of the hitherto unknown potential for botryococcenes to be used to prevent or alleviate such undesirable cosmetic changes in skin.

## Description

### FIELD

The present invention relates to a skin cosmetic composition comprising a botryococcene as an active ingredient, for prevention or alleviation of undesirable cosmetic changes in the skin.

### BACKGROUND

Skin is constantly exposed to stress due to various exogenous and endogenous factors. Such stress factors include sunlight exposure (especially UVB in sunlight; photoaging), air, heat, dryness, wetness, contact with chemical substances such as household surfactants, contact with substances that produce mechanical irritation of the skin, such as polishing agents, side-effects of smoking, alcohol and drugs, physical or psychological stress, biochemical changes in skin due to aging or other factors, hormonal abnormalities, fatigue, acne, malnutrition, diseases and circadian rhythm disruption.

Stress caused by such factors can result in cosmetically undesirable impairment in the outer appearance, clinical and physical properties and physiological and histological function of the skin. Skin becomes fatigued and the biological and mechanical functions of skin tissue become weakened with time, resulting in signs of skin aging.

The undesirable cosmetic changes of note include drying and fine line wrinkles, reduced elasticity, skin sagging, loss of skin moisture, reduced hardness, skin thinning, reduced uniformity of facial color, poor complexion, hyperpigmentation, skin spots, senile moles, red skin spots, rough skin surface texture and mottled complexion. Common symptoms also include loss of hair luster, hair loss and scalp imbalance.

Moreover, when the skin undergoes aging or is chronically exposed to stress in a mild environment, undesirable cosmetic changes may occur that are measurable, though not as significant as those resulting from the direct stress factors mentioned above. Such changes generally include general reduction in tissue and cellular activity, slowdown of cellular replication, reduced protein synthesis, increased proteolysis, blood vessel dilation due to skin blood circulation decrease or congestion, decreased blood infiltration, lower moisture content, accumulation of protein structural and functional errors, undesirable changes in skin barrier, connective tissue adhesion and reduced skin reconstruction or repair ability.

It is known that the major processes that produce notable or measurable cosmetically undesirable changes in skin due to various kinds of stress or aging involve promoting production of reactive oxygen species (ROS) as oxidative stress, and that increased ROS concentration can lead to inflammatory responses, damage to lipids, proteins or DNA and or consequent functional decline of cells, or cell death or tissue destruction. Skin is always exposed to some degree of oxidative stress, and when the intensity of the oxidative stress exceeds the allowable range, or when the mechanism of dealing with oxidative stress in the body is impaired, this results in some manner of undesirable cosmetic change.

It is therefore believed that preventing or eliminating generation of ROS in skin is effective for preventing or alleviating undesirable changes in cosmetic quality of the skin.

One means for eliminating ROS in the body is superoxide dismutase (SOD), a typical enzyme that eliminates ROS, catalyzing reaction for conversion of the reactive oxygen species superoxide to oxygen and hydrogen peroxide. The hydrogen peroxide that is formed is also harmful, but it is eliminated by catalase and glutathione peroxidase (GPx).

Various low molecular compounds such as vitamins, polyphenols, catechins, glutathione, ascorbates, tocopherol, ubiquinone, bilirubin and uric acid may coordinate with antioxidant mechanisms in the body, or may function independently as natural antioxidants. Vitamin B2, for example, acts as a coenzyme with GPx, while polyphenols exhibit SOD-like activity. Carotenoids also function as antioxidants, and can exhibit defensive effects against oxidative stress and related chronic diseases. NPL 1, for example, summarizes reports on the relationship between carotenoids and various chronic diseases such as coronary heart disease, cataract and cancer.

Beauty products comprising such substances with antioxidant activity as active ingredients can therefore be utilized for prevention or alleviation of undesirable cosmetic changes in the skin that are associated with ROS activity. Skin cosmetic compositions using various antioxidants as active ingredients have actually been reported (PTL 1, PTL 2, PTL 3).

The discovery of powerful antioxidant activity by novel compounds or existing compounds with high antioxidant activity has been welcomed in the fields of drugs, foods and cosmetics as widening the range of options for antioxidants that are in high demand.

PTL 4, for example, discloses synthesis of the compound luteolin 6-C-arabinoside as a flavonoid compound that exhibits an excellent antioxidant effect, as well as a method for its production and an antioxidant comprising it as an active ingredient. PTL 5 discloses that epigallocatechin dimer and trimer have activity of eliminating superoxide anion radicals. PTL 6 discloses a novel nitrogen-containing heterocyclic compound or its salt, as well as a therapeutic agent for oxidative stress-related diseases that comprises it.

### [CITATION LIST]

### [PATENT LITERATURE]

[PTL 1] Domestic Re-publication of PCT International Application 01/026670
[PTL 2] Japanese Unexamined Patent Publication No. 2021-095370
[PTL 3] Japanese Patent Public Inspection No. 2017-537091
[PTL 4] Japanese Unexamined Patent Publication No. 2006-265249
[PTL 5] Japanese Unexamined Patent Publication No. 2010-280716
[PTL 6] Japanese Unexamined Patent Publication No. 2007-016011
[PTL 7] Japanese Unexamined Patent Publication HEI No. 7-265059
[PTL 8] Japanese Patent Public Inspection No. 2013-504332
[PTL 9] Japanese Unexamined Patent Publication No. 2013-035791
[PTL 10] Domestic Re-publication of PCT International Application 2012/077801

### [NON PATENT LITERATURE]

[NPL 1] Canfield, et al., (1992) Proc. Soc. Exp. Biol. Med. 200:260
[NPL 2] Kaya, K., Microbiol. Cult. Coll. 26 (1): 1-10, 2010

### SUMMARY

### [TECHNICAL PROBLEM]

It is an object of the present invention to provide a skin cosmetic composition comprising a botryococcene as an active ingredient, for prevention or alleviation of undesirable cosmetic changes in the skin.

### [SOLUTION TO PROBLEM]

Algae belonging to the genus *Botryococcus* are microalgae that utilize light energy to fix carbon dioxide and produce hydrocarbons. Depletion of fossil energy has become a concern in recent years, and carbon dioxide fixation by *Botryococcus* algae is believed to be an effective method for energy acquisition. Another issue is global warming taking place due to increasing carbon dioxide levels resulting from combustion of fossil energy, and the use of *Botryococcus* algae that have the ability to convert carbon dioxide to hydrocarbons holds much promise from the viewpoint of not only utilizing light energy but also reducing carbon dioxide content (PTL 7).

*Botryococcus braunii* (*B. braunii*) a type of colonial alga belonging to the genus *Botryococcus,* is classified as Race-A, Race-B or Race-L, based on the structural features of the hydrocarbons that are produced. Of these, Race-B produces a class of branched triterpenoid hydrocarbons known as botryococcenes and represented as CₙH₂ₙ₋₁₀ (n = 30 to 37) (NPL 2). Triterpenoid hydrocarbons can be used as starting materials for hydrocracking in petroleum refining for production of octanes (gasoline and petrol), kerosene and diesel, for example. Botryococcenes can potentially be converted to biofuels with higher octane numbers (PTL 8).

The use of botryococcenes as cosmetic active ingredients has also been investigated (PTL 9). PTL 9 was filed by the present applicant as a joint applicant. In this publication it was reported that a combined formulation of a botryococcene with a hydrogenated modified botryococcene can be utilized as a humectant that exhibits a more excellent effect than squalane. Since cosmetic uses of botryococcenes can provide much higher value than uses for biofuels, they are powerful incentives for development of botryococcene production techniques by culturing of *Botryococcus braunii.*

Upon further research on cosmetic uses of botryococcenes, the present inventors have demonstrated via multiple tests that botryococcenes inhibit ROS production and its downstream effects which produce undesirable cosmetic changes in skin, and have discovered hitherto unknown potential for botryococcenes to be used to prevent or alleviate such undesirable cosmetic changes in skin.

While PTL 9 describes cosmetic use of botryococcenes as does the present invention, the moisturizing function of skin described in that publication is different and completely unrelated to preventing or alleviating undesirable cosmetic changes in skin associated with ROS, which is the object of the present invention.

The present application thus provides the following inventions.
1. A cosmetic composition comprising a botryococcene as an active ingredient, for prevention or alleviation of undesirable cosmetic changes in the skin.
2. The cosmetic composition according to [1] above, wherein the undesirable cosmetic changes in skin are one or more from among drying and fine line wrinkles, reduced elasticity, skin sagging, loss of skin moisture, reduced hardness, skin thinning, reduced uniformity of facial color, poor complexion, hyperpigmentation, senile moles, red skin spots, rough skin surface texture, mottled complexion, general reduction in tissue and cellular activity, slowdown of cellular replication, reduced protein synthesis, increased proteolysis, blood vessel dilation due to skin blood circulation decrease or congestion, decreased blood infiltration, lower moisture content, accumulation of protein structural and functional errors, undesirable changes in skin barrier, connective tissue adhesion and reduced skin reconstruction or repair ability.
3. The cosmetic composition according to [1] or [2] above, wherein the undesirable cosmetic changes in skin are formation of skin wrinkles or skin spots.
4. The cosmetic composition according to any one of [1] to [3] above, wherein the prevention or alleviation of undesirable cosmetic changes in skin is accomplished by one or more of the actions of botryococcenes selected from among: inhibiting generation of ROS in skin tissue, suppressing production of inflammatory cytokines elicited by ROS, suppressing melanosome phagocytosis by pigment cells elicited by inflammatory cytokines, and suppressing reduction in collagen production or suppressing accelerated production of MMP-1 by fibroblasts, caused by inflammatory cytokines.
5. The cosmetic composition according to [4] above, wherein the ROS in skin tissue is generated by one or more from among: skin sunlight exposure, air, heat, dryness, wetness, contact with chemical substances contact with substances that produce mechanical irritation, side-effects of smoking, alcohol or drugs, physical or psychological stress, biochemical changes in skin due to aging, hormonal abnormalities, fatigue, acne, malnutrition, diseases and circadian rhythm disruption.
6. The cosmetic composition according to [4] above, wherein the ROS in skin tissue is generated by sunlight exposure of the skin.
7. The cosmetic composition according to any one of [1] to [6] above, which further comprises one or more active ingredients that prevent or alleviate undesirable cosmetic changes in the skin.
8. The cosmetic composition according to [7] above, wherein the additional one or more active ingredients are antioxidants.
9. The cosmetic composition according to [8] above, wherein the antioxidant is selected from the group consisting of vitamin A/retinol, vitamin C/ascorbic acid, vitamin E/α-tocopherol, coenzyme Q10, N-acetylcysteine (NAC), S-methylcysteine, glutathione, L-cysteine, D-cysteine, cysteine persulfide, cysteine trisulfide, uric acid, allicin, alliin, allyl disulfide, cyanidin chloride, cyanidin-3,5-diglucoside, anthocyanin, isoflavone, quercetin, catechin, theaflavin, sesamin, sesaminol, resveratrol, curcumin, chlorogenic acid, ferulic acid, 3,4-dihydroxycinnamic acid, β-carotene, lycopene, astaxanthin, zeaxanthin, L-cysteine methyl ester hydrochloride (LCM), L-cysteine ethyl ester hydrochloride (LCE), D-cysteine hydrochloride monohydrate (DCH), D-cysteine methyl ester hydrochloride (DCM), D-penicillamine (DPA), 2-aminoethanethiol (2-AET), L-methionine and L-methionine methyl ester hydrochloride (LMM).
10. The cosmetic composition according to any one of [1] to [9] above, which is in the form of a cosmetic water, cream, latex, gel, aerosol, pack, cleaning agent, bath additive, foundation, powder, lipstick, ointment, poultice, paste, plaster or essence product for skin application.

### BRIEF DESCRIPTION OF DRAWINGS

[Fig. 1]

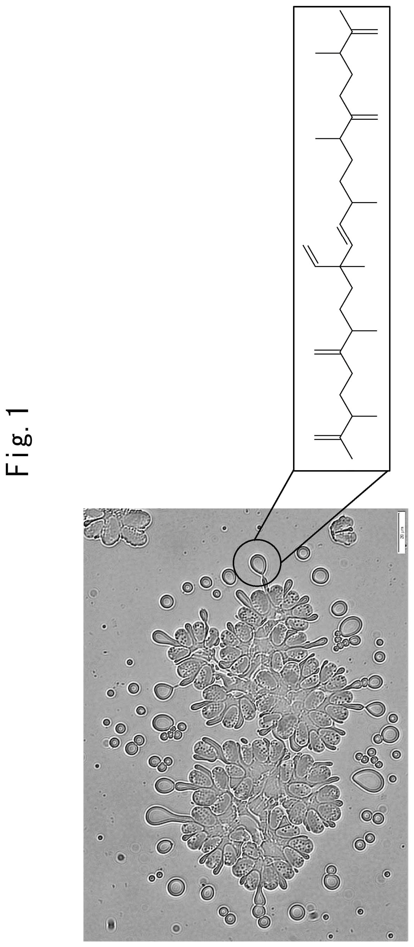

   Fig. 1 shows an optical microscope image of *Botryococcus braunii* secreting botryococcene oil droplets, together with the structural formula for botryococcene.
[Fig. 2]

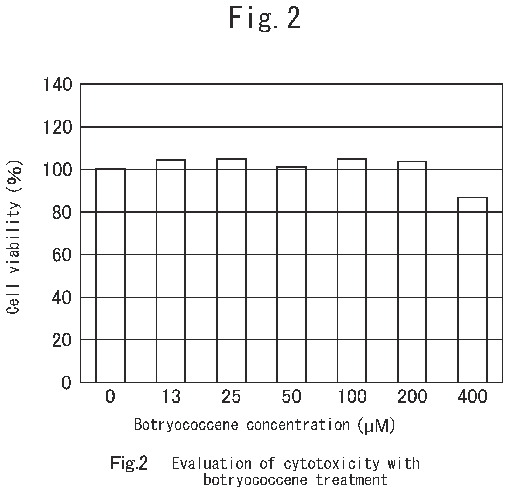

   Fig. 2 shows evaluation of cytotoxicity with botryococcene treatment.
[Fig. 3]

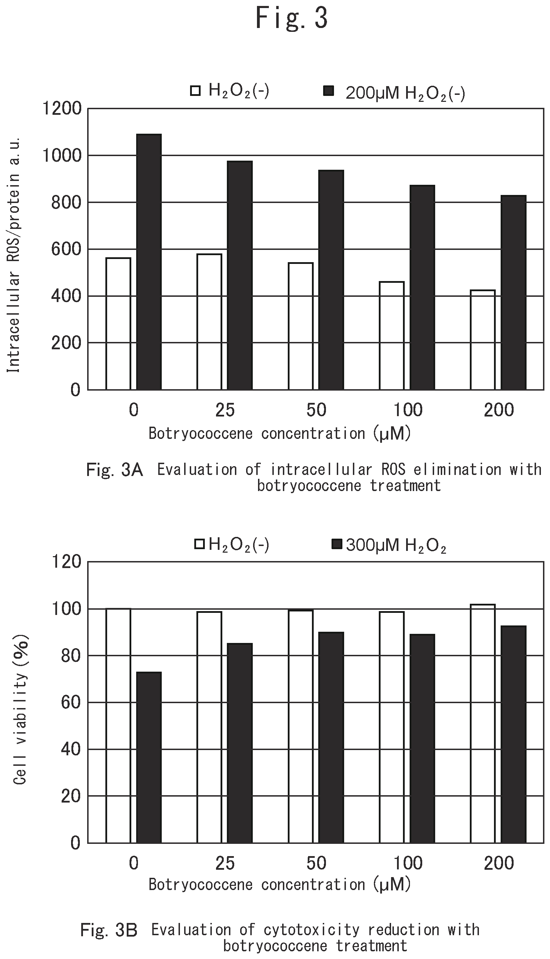

   Fig. 3 shows elimination of intracellular ROS by botryococcene treatment (Fig. 3A) and alleviation of H₂O₂-induced reduction in cell viability by botryococcene treatment (Fig. 3B).
[Fig. 4]

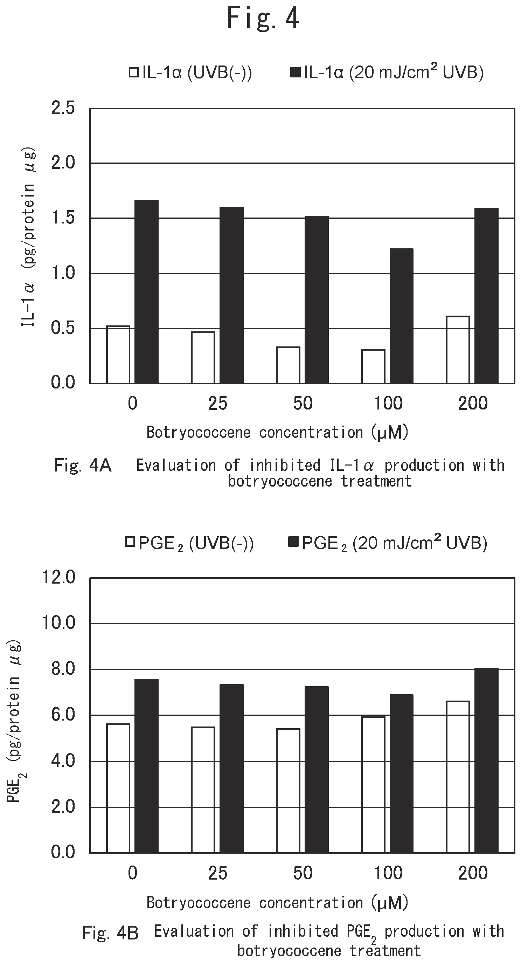

   Fig. 4 shows inhibition of production of inflammatory cytokine IL-1α (Fig. 4A) and PGE₂ (Fig. 4B) by botryococcene treatment.
[Fig. 5]

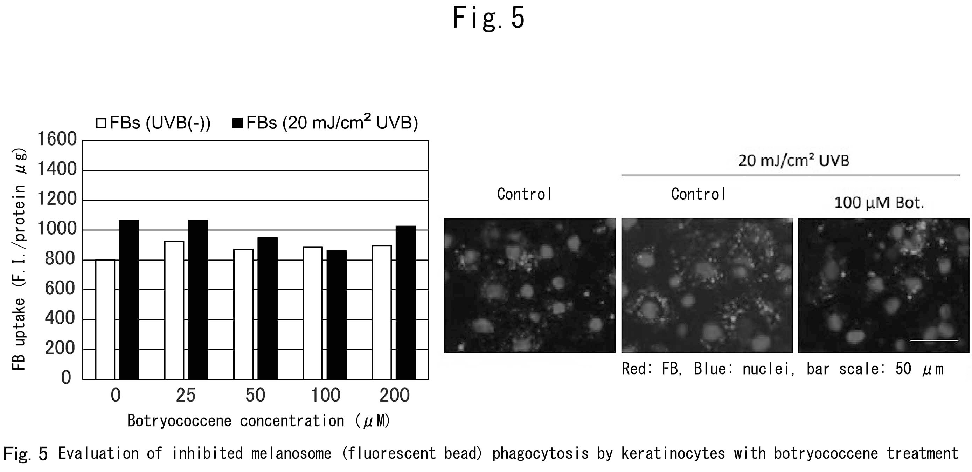

   Fig. 5 shows inhibition of fibroblast phagocytosis of fluorescence beads by botryococcene treatment.
[Fig. 6]

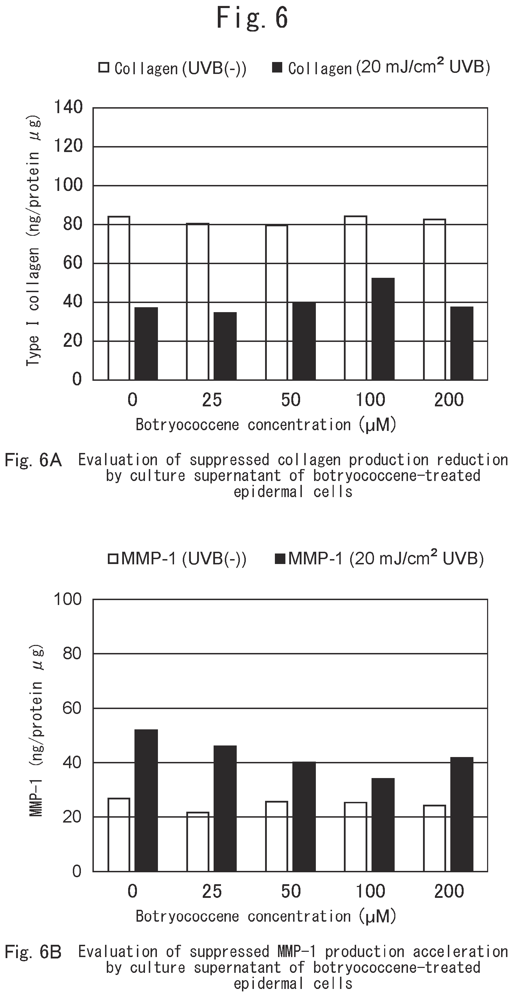

   Fig. 6 shows inhibited reduction in collagen production by epidermal cells (Fig. 6A) and inhibited production of MMP-1 (Fig. 6B) by botryococcene treatment.
[Fig. 7]

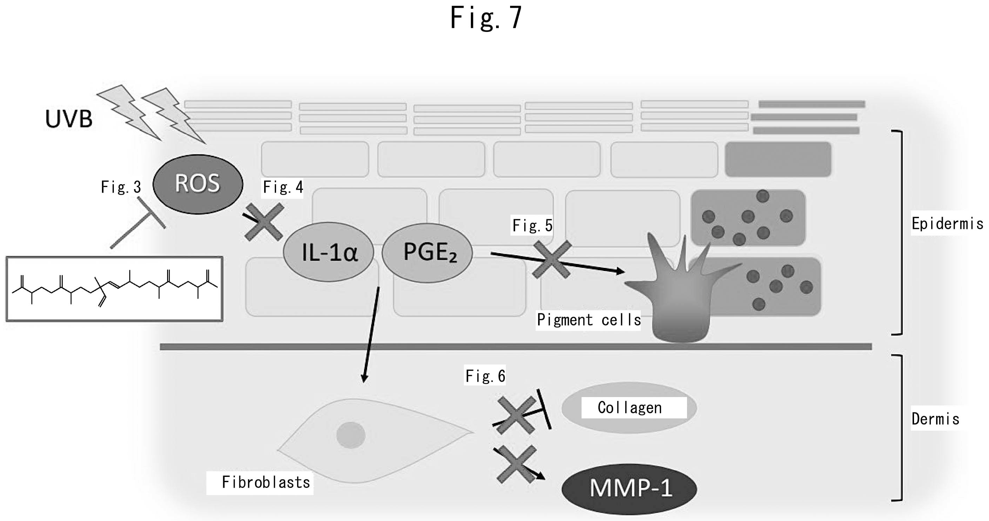

   Fig. 7 is a schematic diagram of the process by which UVB causes undesirable cosmetic changes in skin, highlighting the steps in the process where the effects of botryococcene treatment in Figs. 3 to 6 were observed.

### DESCRIPTION OF EMBODIMENTS

Botryococcenes are a class of branched triterpenoid hydrocarbons represented as CₙH₂ₙ₋₁₀ (n = 30 to 37), produced by some strains of *Botryococcus braunii.* The botryococcene-producing *Botryococcus braunii* race-B has the property of producing a large amount of high-purity botryococcenes, secreting at least 90% of them out of the cells and accumulating them in the colony, and it is therefore useful for mass production of botryococcenes (PTL 10).

Botryococcenes themselves can be directly used as diesel oil for ships and agricultural work vehicles, but existing catalytic cracking can also be used for their conversion to light oil, naphtha, kerosene or gasoline. Moreover, because botryococcenes have excellent moisture retention properties, they have been found to be useful as moisturizing components for addition to cosmetics (PTL 9). Botryococcenes are therefore expected to be useful for a variety of industrial purposes, and the skin cosmetic purpose described herein is an even more promising purpose of use for botryococcenes which further increases their value.

According to the invention, a cosmetic composition comprising a botryococcene as an active ingredient is prepared, and such a cosmetic composition is able to prevent or alleviate undesirable cosmetic changes in the skin.

The effect of botryococcenes of preventing or alleviating undesirable cosmetic changes in skin can be reasonably estimated from test results in which botryococcenes suppress various events that lead to undesirable cosmetic changes in skin, such as production of inflammatory cytokines elicited by increased ROS concentration in the skin, melanosome phagocytosis by pigment cells elicited by inflammatory cytokines, or reduction in collagen production or increased production of MMP-1 by fibroblasts, elicited by inflammatory cytokines, as demonstrated by the Examples below.

Increased ROS concentration in the skin leads to a variety of undesirable cosmetic changes in skin which are mediated by various stress factors in the skin. Such stress factors that cause increased ROS concentration either indirectly or directly include, but are not limited to, sunlight exposure (due to UVB in sunlight; photoaging), air, heat, dryness, wetness, contact with chemical substances such as household surfactants, contact with substances that produce mechanical irritation of the skin, such as polishing agents, side-effects of smoking, alcohol and drugs, physical or psychological stress, biochemical changes in skin due to aging or other factors, hormonal abnormalities, fatigue, acne, malnutrition, diseases and circadian rhythm disruption.

Sunlight exposure is the stress factor of particular importance for the invention. Undesirable cosmetic changes in skin caused by sunlight exposure is generally referred to as "photoaging". Ultraviolet (UV) light, as a component of sunlight, and especially medium-wave UV (UVB, having a wavelength of 290-320 nm) is a major cause of photoaging. While the exposure dose of UVB necessary to cause photoaging is currently unknown, photoaging usually takes place due to repeated exposure to UVB on a level causing transient erythema or sunburn. Clinically, photoaging can be identified by skin roughening, wrinkle formation, discoloration, darkening, sagging, capillary dilation, mole formation, purpura, skin weakening, atrophy, formation of fibrotic pigment deficient areas, premalignant tumors and malignant tumors. Photoaging normally occurs in skin that is habitually exposed to sunlight, such as the face, ears, head, neck and hands.

Undesirable cosmetic changes to be prevented or alleviated by the skin cosmetic composition of the invention include, but are not limited to, drying and fine line wrinkles, reduced elasticity, skin sagging, loss of skin moisture, reduced hardness, skin thinning, reduced uniformity of facial color, poor complexion, hyperpigmentation, senile moles, red skin spots, rough skin surface texture and mottled complexion. Undesirable cosmetic changes that are not noticeable but are measurable, such as general reduction in tissue and cellular activity, slowdown of cellular replication, reduced protein synthesis, increased proteolysis, blood vessel dilation due to skin blood circulation decrease or congestion, decreased blood infiltration, lower moisture content, accumulation of protein structural and functional errors, undesirable changes in skin barrier, connective tissue adhesion and reduced skin reconstruction or repair ability, are also expected to be prevented or alleviated by the skin cosmetic composition of the invention.

Undesirable cosmetic changes that are particularly to be prevented or reduced by the skin cosmetic composition of the invention are the formation of skin wrinkles or skin spots. An increase in ROS concentration in skin mainly promotes production of the inflammatory cytokine IL-1α and PGE₂, the activity of which promotes melanosome phagocytosis by skin epidermal cells to produce skin spots, while also inhibiting collagen production by dermal fibroblasts or promoting MMP-1 production, thus producing skin wrinkles. In the Examples of the present application it is demonstrated that the skin cosmetic composition of the invention exhibits an inhibiting effect at points throughout the pathway from increase in ROS concentration to formation of skin spots or wrinkles (Fig. 7).

The skin cosmetic composition of the invention may be prepared in any suitable dosage form that is to be applied to skin for prevention or alleviation of undesirable cosmetic changes. Without being restrictive, the dosage form may be a cosmetic water, cream, latex, gel, aerosol, pack, cleaning agent, bath additive, foundation, powder, lipstick, ointment, poultice, paste, plaster or essence product. Determining the form in which the skin cosmetic composition of the invention is to be prepared, and designing a component composition suitable for forming the desired dosage form, are within the scope of ordinary optimization and analysis of formulation conditions by a person skilled in the art.

The botryococcene content of the skin cosmetic composition of the invention sufficient to achieve a desired effect can be appropriately determined by considering the dosage form, method and dosage of the composition, the desired cosmetic effect and the types or extents of undesirable cosmetic changes, as well as by preliminary testing ordinarily carried out by a person skilled in the art of cosmetics. According to one aspect, the skin cosmetic composition of the invention comprises a botryococcene at a concentration of 0.001 to 20 wt%, more preferably it comprises a botryococcene at a concentration of 0.001 to 15 wt%, even more preferably it comprises a botryococcene at a concentration of 0.001 to 10 wt%, yet more preferably it comprises a botryococcene at a concentration of 0.01 to 3 wt%, and even yet more preferably it comprises a botryococcene at a concentration of 0.01 to 2 wt%. The botryococcene concentration, for the purpose of the Examples described herein, is the concentration applied to *in vitro* cultured cells, and it does not define the range of the botryococcene content in the skin cosmetic composition of the invention.

The skin cosmetic composition of the invention may also comprise one or more additional active ingredients in addition to a botryococcene. Such additional active ingredients may aid in preventing or alleviating, or in additively or synergistically reinforcing, unwanted undesirable cosmetic changes in skin by the skin cosmetic composition of the invention. For example, the skin cosmetic composition of the invention may comprise an antioxidant as one or more additional active ingredients.

Antioxidants to be added to the skin cosmetic composition of the invention include, but are not limited to, vitamin A/retinol, vitamin C/ascorbic acid, vitamin E/α-tocopherol, coenzyme Q10, N-acetylcysteine (NAC), S-methylcysteine, glutathione, L-cysteine, D-cysteine, cysteine persulfide, cysteine trisulfide, uric acid, allicin, alliin, allyl disulfide, cyanidin chloride, cyanidin-3,5-diglucoside, anthocyanin, isoflavone, quercetin, catechin, theaflavin, sesamin, sesaminol, resveratrol, curcumin, chlorogenic acid, ferulic acid, 3,4-dihydroxycinnamic acid, β-carotene, lycopene, astaxanthin, zeaxanthin, L-cysteine methyl ester hydrochloride (LCM), L-cysteine ethyl ester hydrochloride (LCE), D-cysteine hydrochloride monohydrate (DCH), D-cysteine methyl ester hydrochloride (DCM), D-penicillamine (DPA), 2-aminoethanethiol (2-AET), L-methionine and L-methionine methyl ester hydrochloride (LMM).

### EXAMPLES

In the following Examples, the effects of botryococcenes added to the skin cosmetic composition of the invention are demonstrated in an *in vitro* model of various molecular biological events that lead to undesirable cosmetic changes in skin.

### Cell culturing

Normal human epidermal keratinocytes (NHEKs) were cultured in HuMedia-KG2 (KG2) medium containing 10% fetal bovine serum, 1% antibiotic and 1% human epidermal keratinocyte growth supplement, in a 37°C, 5% CO₂ incubator.

Normal human skin fibroblasts (NHDFs) were cultured in DMEM with addition of 10% fetal bovine serum, 1% antibiotic and 1% human epidermal keratinocyte growth supplement, in a 37°C, 5% CO₂ incubator.

### Measurement of cytotoxicity with botryococcene treatment

NHEK cells were seeded on a 96-well plate at a density of 2.5 × 10⁴ cells/well in KG2 medium, and incubated for 24 hours. The supernatant was aspirated, the medium was exchanged with HuMedia-KB2 (KB2) medium containing 0, 12.5, 25, 50, 100, 200 and 400 µM botryococcene, and the mixture was incubated for 24 hours. After culturing, the cells were washed with PBS, the medium was exchanged with KB2 medium containing 0.003% Neutral Red, and culturing was carried out for 2 hours. The Neutral Red in the cells was extracted with 1 M HCl in 30% MeOH. The absorbance at 550 nm was measured with a microplate reader.

The test results are shown in Fig. 2. No change in cell viability was observed with a botryococcene concentration of 0 to 200 µM, but a notable reduction was observed with 400 µM. Based on these results, 200 µM was used as the maximum botryococcene treatment concentration in the subsequent test.

### Example 1. Intracellular reactive oxygen species removal effect of botryococcene

NHEK cells were seeded on a 96-well plate at a density of 2.5 × 10⁴ cells/well in KG2 medium, and incubated for 24 hours. The supernatant was removed, the medium was exchanged with KB2 medium containing 0, 25, 50, 100 and 200 µM botryococcene, and incubation was carried out for 24 hours. After culturing, the cells were washed with PBS, the medium was exchanged with HBSS buffer containing 200 µM H₂O₂, and culturing was continued for 1 hour. The supernatant was removed and culturing was carried out for 30 minutes in HBSS buffer containing 20 µM DCFH-DA. The fluorescence intensity was measured with a microplate reader (Emission = 485 nm, Excitation = 530 nm). The cells were then dissolved in 50 µL of PBS containing 0.5% Triton^{R} X-100, the protein level was quantified, and the fluorescence intensity per unit protein was normalized.

The test results are shown in Fig. 3A. The intracellular ROS concentration increased with 200 µM H₂O₂ treatment. Both the intracellular ROS concentrations that increased by H₂O₂ treatment and the intracellular ROS concentrations without H₂O₂ treatment were observed to decrease in a concentration-dependent manner with addition of a botryococcene.

### Example 2. Effect of botryococcene against cytotoxicity induced by addition of H₂O₂

NHEK cells were seeded on a 96-well plate at a density of 2.5 × 10⁴ cells/well in KG2 medium, and incubated for 24 hours. The supernatant was aspirated, the medium was exchanged with KB2 medium containing 0, 25, 50, 100 and 200 µM botryococcene, and incubation was carried out for 24 hours. After culturing, the cells were washed for 1 hour with HBSS containing PBS and 300 µM H₂O₂, and the cells were then incubated for 24 hours in KB2 medium. After incubation, the cells were washed with PBS, the medium was exchanged with KB2 medium containing 0.003% Neutral Red, and culturing was carried out for 2 hours. After culturing, the cells were washed with PBS. The Neutral Red in the cells was extracted with 1 M HCl in 30% MeOH. The absorbance at 550 nm was measured with a microplate reader.

The test results are shown in Fig. 3B. The reduction in cell viability by 300 µM H₂O₂ treatment was attributed to increased intracellular ROS concentration. The reduction in cell viability by H₂O₂ treatment was restored by addition of botryococcene, in a concentration-dependent manner.

### Example 3. Effect of botryococcene against accelerated IL-1α and PGE₂ production induced by UVB irradiation

NHEK cells were seeded on a 96-well plate at a density of 2.5 × 10⁴ cells/well in KG2 medium, and incubated for 24 hours. The supernatant was aspirated, the medium was exchanged with KB2 medium containing 0, 25, 50, 100 and 200 µM botryococcene, and culturing was continued for 24 hours, after which the cells were washed with PBS and the medium was exchanged with HBSS. The cells were then irradiated with UVB (280 to 320 nm, 20 mJ/cm²) and cultured for 24 hours in fresh KB2 medium. The IL-1α and PGE₂ expression levels in the culture supernatants were measured using an ELISA measuring kit. The cells were then dissolved in 50 µL of PBS containing 0.5% Triton^{R} X-100, the protein level was quantified, and the IL-1α and PGE₂ production levels per unit protein were calculated.

The test results are shown in Fig. 4. UVB irradiation notably increases the concentrations of the inflammatory cytokines IL-1α and PGE₂, thereby reproducing the skin inflammatory response to UVB irradiation, in which UVB irradiation leads to oxidative stress on cells and induces inflammatory cytokine production. Botryococcene addition inhibited all of the IL-1α and PGE₂ production induced by UVB irradiation in a concentration-dependent manner up to 100 µM. Induction of IL-1α and PGE₂ production was not inhibited under conditions with a botryococcene concentration of 200 µM. The amounts of IL-1α and PGE₂ produced under non-UVB-irradiated conditions were also found to be reduced by botryococcene in a concentration-dependent manner up to 100 µM for IL-1α and 50 µM for PGE₂.

### Example 4. Effect of botryococcene against accelerated fluorescent bead phagocytosis induced by UVB irradiation

NHEK cells were seeded on a 96-well plate at a density of 2.5 × 10⁴ cells/well in KG2 medium, and incubated for 24 hours. The supernatant was aspirated, the medium was exchanged with KB2 medium containing 0, 25, 50, 100 and 200 µM botryococcene, and culturing was continued for 24 hours, after which the cells were washed with PBS and the medium was replaced with HBSS. The cells were then irradiated with UVB (280 to 320 nm, 20 mJ/cm²) and cultured for 24 hours in fresh KB2 medium. After replacing the medium with of KB2 medium containing FluoSpheres^{™} and allowing the cells to stand for 4 hours, they were dissolved with 50 µL of PBS containing 0.5% Triton^{R} X-100 and the fluorescence of the cell lysate was measured with a microplate reader. The fluorescence of the cell lysate was detected with a fluorescence reader at an excitation/luminous wavelength of 535 nm/590 nm. The cells were then dissolved in 50 µL of PBS containing 0.5% Triton^{R} X-100, the protein level was quantified, and the fluorescence intensity per unit protein was normalized.

The test results are shown in Fig. 5. Phagocytosis of FluoSpheres^{™} by NHEK cells constitutes *in vitro* reproduction of a system in which pigment cells that have been subjected to UVB irradiation stimulation (via ROS and inflammatory cytokines) phagocytose melanosomes and accumulate them in the cells, forming skin spots. Addition of botryococcene inhibited fluorescent bead phagocytosis by UVB irradiation in a concentration-dependent manner up to 100 µM. Under conditions with a botryococcene concentration of 200 µM, the effect of inhibiting fluorescent bead phagocytosis was less prominent than with 50 µM or 100 µM.

### Example 5. Effect of botryococcene against reduced type I collagen production and accelerated MMP-1 production induced by UVB irradiation

NHEK cells were seeded on a 96-well plate at a density of 2.5 × 10⁴ cells/well in KG2 medium, and incubated for 24 hours. The supernatant was aspirated, the medium was exchanged with KB2 medium containing 0, 25, 50, 100 and 200 µM botryococcene, and culturing was continued for 24 hours, after which the cells were washed with PBS and the medium was replaced with HBSS. The cells were then irradiated with UVB (280 to 320 nm, 20 mJ/cm²) and cultured for 24 hours in fresh KB2 medium. After culturing, the supernatant (K-CM) was collected. NHDF cells were seeded on a 96-well plate at a density of 2.0 × 10⁴ cells/well in DMEM medium, and incubated for 24 hours. After culturing, K-CM was added and culturing was continued for 24 hours. The supernatant was provided for ELISA analysis of type I collagen and MMP-1 production. The cells were then dissolved in 50 µL of PBS containing 0.5% Triton^{R} X-100, and the protein level was quantified with a BCA Protein Assay Kit (product of Thermo Fisher Scientific), calculating the type I collagen and MMP-1 production per unit protein.

The test results are shown in Fig. 6. This test constitutes *in vitro* reproduction of a system in which epidermal cells that have been subjected to UVB irradiation produce inflammatory cytokines, with inhibition of collagen production and acceleration of MMP-1 production by fibroblasts due to the inflammatory cytokines, thereby forming skin wrinkles. The culture supernatant of UVB-irradiated NHEK cells showed a powerful collagen production-inhibiting effect and an MMP-1 production-accelerating effect, which was attributed to inflammatory cytokines being released into the medium by the UVB-irradiated NHEK cells. Addition of botryococcene inhibited the reduction in collagen production and acceleration of MMP-1 production in a concentration-dependent manner up to 100 µM. Under conditions with a botryococcene concentration of 200 µM, the inhibiting effect was less prominent than with 50 µM or 100 µM.

## Claims

1. A cosmetic composition comprising a botryococcene as an active ingredient, for prevention or alleviation of undesirable cosmetic changes in the skin.

2. The cosmetic composition according to claim 1, wherein the undesirable cosmetic changes in skin are one or more from among drying and fine line wrinkles, reduced elasticity, skin sagging, loss of skin moisture, reduced hardness, skin thinning, reduced uniformity of facial color, poor complexion, hyperpigmentation, senile moles, red skin spots, rough skin surface texture, mottled complexion, general reduction in tissue and cellular activity, slowdown of cellular replication, reduced protein synthesis, increased proteolysis, blood vessel dilation due to skin blood circulation decrease or congestion, decreased blood infiltration, lower moisture content, accumulation of protein structural and functional errors, undesirable changes in skin barrier, connective tissue adhesion and reduced skin reconstruction or repair ability.

3. The cosmetic composition according to claim 1, wherein the undesirable cosmetic changes in skin are formation of skin wrinkles or skin spots.

4. The cosmetic composition according to claim 1, wherein the prevention or alleviation of undesirable cosmetic changes in skin is accomplished by one or more of the actions of botryococcenes selected from among: inhibiting generation of reactive oxygen species (ROS) in skin tissue, suppressing production of inflammatory cytokines elicited by ROS, suppressing melanosome phagocytosis by pigment cells elicited by inflammatory cytokines, and suppressing reduction in collagen production or suppressing accelerated production of MMP-1 by fibroblasts, caused by inflammatory cytokines.

5. The cosmetic composition according to claim 4, wherein the ROS in skin tissue is generated by one or more from among: skin sunlight exposure, air, heat, dryness, wetness, contact with chemical substances contact with substances that produce mechanical irritation, side-effects of smoking, alcohol or drugs, physical or psychological stress, biochemical changes in skin due to aging, hormonal abnormalities, fatigue, acne, malnutrition, diseases and circadian rhythm disruption.

6. The cosmetic composition according to claim 4, wherein the ROS in skin tissue is generated by sunlight exposure of the skin.

7. The cosmetic composition according to claim 1, which further comprises one or more active ingredients that prevent or alleviate undesirable cosmetic changes in the skin.

8. The cosmetic composition according to claim 7, wherein the additional one or more active ingredients are antioxidants.

9. The cosmetic composition according to claim 8, wherein the antioxidant is selected from the group consisting of vitamin A/retinol, vitamin C/ascorbic acid, vitamin E/α-tocopherol, coenzyme Q10, N-acetylcysteine (NAC), S-methylcysteine, glutathione, L-cysteine, D-cysteine, cysteine persulfide, cysteine trisulfide, uric acid, allicin, alliin, allyl disulfide, cyanidin chloride, cyanidin-3,5-diglucoside, anthocyanin, isoflavone, quercetin, catechin, theaflavin, sesamin, sesaminol, resveratrol, curcumin, chlorogenic acid, ferulic acid, 3,4-dihydroxycinnamic acid, β-carotene, lycopene, astaxanthin, zeaxanthin, L-cysteine methyl ester hydrochloride (LCM), L-cysteine ethyl ester hydrochloride (LCE), D-cysteine hydrochloride monohydrate (DCH), D-cysteine methyl ester hydrochloride (DCM), D-penicillamine (DPA), 2-aminoethanethiol (2-AET), L-methionine and L-methionine methyl ester hydrochloride (LMM).

10. The cosmetic composition according to claim 1, which is in the form of a cosmetic water, cream, latex, gel, aerosol, pack, cleaning agent, bath additive, foundation, powder, lipstick, ointment, poultice, paste, plaster or essence product for skin application.
